Europäisches Patentamt

European Patent Office

Office européen des brevets

(11) Veröffentlichungsnummer: **0 017 930**
**B1**

(12) **EUROPÄISCHE PATENTSCHRIFT**

(45) Veröffentlichungstag der Patentschrift:
07.10.81

(21) Anmeldenummer: 80101955.5

(22) Anmeldetag: 11.04.80

(51) Int. Cl.³: **A 61 F 1/03**

(54) **Hüftkopfkappe für Endoprothese.**

(30) Priorität: 11.04.79 DE 2914737

(43) Veröffentlichungstag der Anmeldung:
29.10.80 Patentblatt 80/22

(45) Bekanntmachung des Hinweises auf die Patenterteilung:
07.10.81 Patentblatt 81/40

(84) Benannte Vertragsstaaten:
AT BE CH FR GB LI NL SE

(56) Entgegenhaltungen:
DE-C-876 739
GB-A-718 935

MEDIZINISCH-ORTHOPÄDISCHE TECHNIK, Band 95,
Nr. 6, November/Dezember 1975 Stuttgart
D. GEDULDIG et al. «Welche Aussicht hat die Biokeramik als Implantatmaterial in der Orthopädie?»
Seiten 138 bis 143

(73) Patentinhaber: **Feldmühle Aktiengesellschaft,
Fritz-Vomfelde-Platz 4, D-4000 Düsseldorf 11 (DE)**

(72) Erfinder: **Prüssner, Peter, Ing.grad., Waldstrasse 38,
D-6057 Dietzenbach (DE)**
Erfinder: **Zichner, Ludwig, Dr., Stieglitzstrasse 16,
D-6078 Neu-Isenburg - Grafenbruch (DE)**
Erfinder: **Dörre, Erhard, Dr. Dipl.-Phys., Tagweg 14,
D-7310 Plochingen (DE)**

(74) Vertreter: **Uhlmann, Hans, Dr. rer.nat., Dipl.-Chem.,
Gladbacher Strasse 189, D-4060 Viersen 1 (DE)**

Hüftkopfkappe für Endoprothese

Die Erfindung betrifft eine Hüftkopfkappe als Endoprothese, die aussen im wesentlichen Kuppelform aufweist und zur teilweisen Aufnahme des Femurs eine im wesentlichen rotationssymmetrische Ausnehmung mit zum Schenkelhals weisender Öffnung besitzt. Der Einsatz von Kugelkappen am proximalen Femurende ist durch eine Vielzahl von Literaturstellen bekannt und beispielsweise in GB-A-720 092, 764 600, US-A-3 521 302, 3 925 824, DE-A-2 724 040, 2 512 407, 2 535 649, 2 422 617, 2 318 459, DE-C-1 164 019, 923 383, 876 739, FR-A-2 094 904, sowie der Zeitschrift «Medizinisch-Orthopädische Technik», Nr. 6, 1975 beschrieben. Dem gesamten Stand der Technik ist dabei eines gemeinsam, dass eine vollständige Bearbeitung des Femurkopfes erfolgen muss. Durch diese Bearbeitung werden meist die Versorgungsgefässe des Schenkelhalses weitgehend zerstört, d.h., die Versorgung des Knochenstumpfes ist nicht mehr gewährleistet, wodurch es zu Nekrosen des Stumpfes kommt und als Folge davon eine Lockerung der Kugelkappe auftritt. Eine weitere grosse Schwierigkeit bei den bisherigen Befestigungen der Kugelkappen war die Verhinderung der Drehbewegung.

So wurde vorgeschlagen, vertikale Rippen oder Riefen in den Kugelhohlraum einzubringen — GB-A-720 092, US-A-3 925 824 — oder die Befestigung durch Befestigungsstifte vorzunehmen, die gleichzeitig der Primärverankerung dienen sollen — US-A-3 521 302, DE-C-1 164 019, DE-C-923 383, DE-C-876 739. Statt der Befestigung durch Stifte oder Rippen wird gemäss der FR-A-2 094 904 vorgeschlagen, eine Blechkappe mit einem Zahnkranz zu versehen und diese Zähne entlang des nicht resezierten Kranzes des Femurkopfes in das Knochengewebe einzuschlagen.

Aus der DE-A-2 318 459 und der Zeitschrift «Medizinisch-Orthopädische Technik», Nr. 6, 1975» ist bekannt, die Hüftkopfkappe mit einem Innengewinde oder einem Innenvielkant zu versehen, so dass sie aufgeschraubt oder aufgesteckt werden kann. Beide Befestigungsarten sind jedoch nicht nur äusserst aufwendig, weil sie ein völliges Freilegen des Femurendes und damit eine schwere Operation bedingen, sie führen auch auf Grund der Tatsache, dass die Achse der Hüftkopfkappe mit der Schenkelhalsmittelachse zusammenfällt, zu einer weitgehenden Zerstörung der Gefässversorgung.

Bei weiteren Kappen ist die Befestigung mit Knochenzement vorgesehen. Durch die bei der Aushärtung auftretende Wärmebelastung wird jedoch das Gewebe stark geschädigt. Auch sind toxische Erscheinungen durch das Monomer des Knochenzementes möglich.

Aufgabe der vorliegenden Erfindung ist deshalb die Schaffung eines Gelenkflächenersatzes der eingangs genannten Art für das proximale Femurende, der nur eine minimale Resektion erforderlich macht, um bei einem Versagen alle Rückzugsmöglichkeiten, wie Arthrodese oder konventionelle Total-Endoprothese offenzulassen, der ferner bei vollständiger Erhaltung des Schenkelhalses nur die tribologischen, nicht aber die biomechanischen Verhältnisse ändert, des weiteren eine gute Gefässversorgung gewährleistet, eine feste Primärfixation ohne Knochenzement sowie das Einwachsen von Knochenstrukturen zur zusätzlichen Langzeitverankerung ermöglicht und einfach zu implantieren ist.

Diese Aufgabe wird erfindungsgemäss dadurch gelöst, dass die Rotationsachse des Hohlraums der Hüftkopfkappe im Einbauzustand etwa parallel zur Femurmittelachse verläuft, und dass der Hohlraum den resezierten Hüftkopfstumpf um mehr als 180 Grad umgreift und eine U-förmig gestaltete, zum Schenkelhals weisende Öffnung besitzt. Die Hüftkopfkappe weist damit in der Seitenansicht gesehen die Form einer Badekappe auf, wobei die bei der Badekappe vorhandene, das Gesicht freilassende Öffnung dem Schenkelhals zugewandt ist. Die wichtigen Gefässe, die den Stumpf versorgen, werden dadurch nicht beschädigt. Da die U-förmige Öffnung in der Hüftkopfkappe weniger als die Hälfte der Kuppelfläche ausmacht, wird der Zylinderstumpf um mehr als 180 Grad durch die Hüftkopfkappe umfasst, d.h. dass die Hüftkopfkappe fest verankert, so dass sie nicht abkippen kann. Der Umfassung des Zylinderstumpfes um mehr als 180 Grad kommt erhebliche Bedeutung zu, da es hier erstmalig mit einfachen Mitteln gelingt, eine Hüftkopfkappe fest zu verankern, ohne dass Knochenzement zu Hilfe genommen werden muss oder dass zusätzliche mechanische Massnahmen ergriffen werden müssen.

Da die Mittelachse des kuppelförmigen Hohlraumes im wesentlichen parallel zur Femurmittelachse verläuft, wird die Krafteinleitung in den Gelenkflächenersatz günstiger, so dass die Kappe ohne zusätzliche Fixierungsmittel, also insbesondere ohne Knochenzement, rotationsstabil und absolut kippsicher verankerbar ist. Die Versorgung des Knochens bleibt auf Grund der U-förmigen Öffnung erhalten. Es ist nur eine minimale Resektion erforderlich; damit ist jederzeit eine Nachoperation möglich, bei der z.B. konventionelle Endoprothesen eingesetzt werden können.

Gemäss einer besonders bevorzugten Ausgestaltung der Erfindung hat der kuppelförmige Hohlraum in der Hüftkopfkappe Zylinderform; zweckmässig ist auch die Ausgestaltung als Kegelstumpf.

Durch die Ausgestaltung des kuppelförmigen Hohlraumes als Zylinder bzw. Kegelstumpf, also als rotationssymmetrischer Körper, ergibt sich eine erhebliche Vereinfachung bei der Bearbeitung des Femurendes. Durch Einbringung einer Zentrierbohrung in den Hüftkopf ist die Resektion des Hüftkopfes mittels einer oszillierenden Säge möglich. Dadurch können wesentlich engere Toleranzen eingehalten werden. Des weiteren vereinfacht sich die Bearbeitung der im hinteren Operationsbereich liegenden Stellen des Hüftkopfes. Die Hüftkopfkappe wird, wenn sie aus einem biokompatiblen Material besteht, das im Körper weder resorbiert noch angegriffen wird, gut einheilen, dazu ist jedoch unabdingbare Voraussetzung, dass der Einheilungsprozess ohne Störung durch Bewegung zwischen dem Knochenstumpf und der Hüftkopfkappe ablaufen kann. Es muss also eine sogenannte Primärfixation vorhanden sein, derart, dass die Hüftkopfkappe auch bei einer eventuellen Körperbewegung sich nicht auf dem Femurende bewegen kann.

Beim gesamten Stand der Technik muss der Femurkopf allseitig bearbeitet werden, wodurch die Gefässversorgung des Knochenstumpfes weitgehend zerstört wird. Es ist deshalb extrem wichtig, dass bei der Erfindung der Knochenstumpf in Richtung des Schenkelhalses, von dem die Versorgung ausgeht, nicht beschädigt wird, d.h. in diesem Bereich nicht von der Hüftkopfkappe umfasst wird, also auch nicht bearbeitet werden muss.

Die Verdrehsicherheit der Hüftkopfkappe wird dabei durch die nicht abgefrästen Anschläge des Hüftkopfes gewährleistet, an denen die Enden der Öffnung der Kappe anliegen.

Eine bevorzugte Ausgestaltung der Erfindung sieht vor, dass die Hüftkopfkappe mittels Presssitz mit dem Femur verbunden ist. Die Befestigung mittels Presssitz sorgt für eine ausgezeichnete Primärfixation, ohne dass Knochenzement erforderlich ist. Wesentliche Voraussetzung für den Einbau einer Hüftkopfkappe ist dabei jedoch, dass enge Toleranzen zwischen Femurkopf und Ausnehmung der Hüftkopfkappe eingehalten werden. Lediglich wenn die genaue Einhaltung der Toleranzen gewährleistet ist, ist das Einsetzen einer Hüftkopfkappe mittels Presssitz möglich, d.h. dass Voraussetzung dafür die mechanische Bearbeitung des Hüftkopfes ist, also die Resektion mittels eines genau geführten Werkzeuges erfolgt. Als besonders zweckmässig dafür hat sich eine oszillierende Hohlfräse erwiesen, wobei je nach Form der Hohlfräse die resezierten Teile Zylinder- oder Kegelform aufweisen, die Resektion aber so erfolgt, dass in jedem Fall zum Schenkelhals des Femurs als auch zum Fuss der Hüftkopfkappe keine Bearbeitung des Knochens stattfindet, um die Gefässversorgung nicht zu beeinträchtigen. Die Hohlfräse ist zweckmässig mit einem Führungsstift ausgestattet, der in eine Bohrung eingreift, die vor Ansetzen der Hohlfräse in das Femurende eingebracht wurde. Diese Bohrung erstreckt sich parallel zur Femurmittelachse, wodurch sich automatisch nach der Resektion des Knochens eine Form ergibt, die sich ebenfalls parallel zur Femurmittelachse erstreckt. Die wesentlichen, der Gefässversorgung dienenden Bereiche des Femurendes werden dadurch nicht beschädigt, ausserdem wird die Krafteinleitung bei Belastung des Knochens wesentlich verbessert, da die Hüftkopfkappe durch diese Anordnung direkt vertikal belastet wird, also nicht mehr wie bisher üblich, unter einem Winkel, was einer der Gründe war, die zur Lockerung von Hüftkopfkappen führten.

Vorteilhafterweise ist die Innenfläche der Hüftkopfkappe mit Nuten und/oder Rippen versehen, die sich zweckmässig parallel und/oder senkrecht zur Zylinder- bzw. Konusmittelachse erstrecken. Diese Nuten oder Rippen bieten den für die Sekundärverankerung erforderlichen Raum, also den Platz, in den die Knochenstrukturen einwachsen können, um so zu einer festen Verankerung der Hüftkopfkappe zu führen. Wegen der auftretenden Belastungen empfiehlt es sich jedoch, diese Nuten bzw. Rippen nur in den Seitenbereichen und nicht im Stirnbereich der Hüftkopfkappe anzuordnen, weil im Stirnbereich die grössere Belastung auftritt und dadurch eine Schwächung der Hüftkopfkappe in diesem Bereich vermieden werden muss.

Besonders zweckmässig ist es, wenn die Innenfläche der Hüftkopfkappe eine rauhe Oberfläche besitzt. Diese rauhe Oberfläche kann bereits beim Bearbeitungsvorgang der Ausnehmung in die Hüftkopfkappe eingebracht werden. Es ist jedoch auch möglich, sie durch nachträgliches Einbringen einer weiteren Schicht vorzusehen, wobei dieses Einbringen einer weiteren Schicht gegebenenfalls durch Flamm- oder Plasmaspritzen erfolgen kann. Besonders wesentlich ist die rauhe Oberfläche im Bereich der Seiten der Hüftkopfkappe, weil sie ebenso wie die Rippen bzw. Nuten eine gute Einwachsmöglichkeit für den Knochen bietet. Eine Schwächung der Stirnfläche tritt dabei nicht auf. Vorteilhafterweise ist die Innenfläche der Hüftkopfkappe mit Poren versehen, deren Grösse mehr als $80\,\mu m$ beträgt. Poren einer Grösse von unter $60\,\mu m$ bieten erfahrungsgemäss für den einwachsenden Knochen keinen Halt mehr. Poren einer Grösse von unter $80\,\mu m$ nur eine geringfügige Befestigung. Eine wirksame echte Befestigung tritt vorzugsweise erst ab einer Porengrösse von $100\,\mu m$ auf.

Als Material für die Hüftkopfkappe können praktisch alle biokompatiblen Werkstoffe eingesetzt werden. Es muss jedoch darauf geachtet werden, dass sie beim Zusammenarbeiten mit der Pfanne keinen Abrieb verursachen. So sind Kombinationen von Metallhüftkopfkappe mit Polyäthylenpfanne möglich, ebenso wie Kohlenstoff, Cobalt-Basislegierungen, sowie Spezialstähle und Titan sich bewährt haben. Auf Grund der extrem niedrigen Abriebwerte, die praktisch beinahe Null liegen und der ausgezeichneten Körperverträglichkeit wird jedoch Oxidkeramik bevorzugt.

Gemäss einer sehr zweckmässigen Ausgestaltung der Erfindung besteht daher die Hüftkopfkappe aus hochreiner Sinteroxidkeramik. Unter dem Begriff «hochrein» ist dabei zu verstehen, dass die Sinteroxidkeramik eine Reinheit von über 95% aufweist, wobei unter dem Begriff «Sinteroxidkeramik» im Sinne der vorstehenden Ausführungen die gesinterten Metalloxide von Zirkon, Titan und insbesondere Aluminium, sowie deren Gemische zu verstehen sind. Gemäss einer bevorzugten Ausgestaltung der Erfindung besteht die Hüftkopfkappe aus gesintertem Aluminiumoxid mit einer Dichte gleich oder grösser 3,92 g/cm³, einer Porosität gleich oder kleiner 2%, einer Wasseraufnahme gleich oder kleiner 0,01%, einer Reinheit gleich oder grösser 99,7% $Al_2O_3$, einer Vickershärte (P=2M) gleich oder grösser 22 000 N/mm², einer mittleren Korngrösse gleich oder kleiner $10\,\mu$m, einer mittleren Biegefestigkeit gleich oder grösser 300 N/mm², einer Druckfestigkeit gleich oder grösser 4000 N/mm² und einer Zugfestigkeit gleich oder grösser 160 N/mm².

Ein derartiges Material ergibt die erforderliche hohe Sicherheit für eine Endoprothese über Jahrzehnte hinaus, wobei gerade durch die Kombination der hohen Dichte mit der niedrigen mittleren Korngrösse und der hohen Reinheit des gesinterten Aluminiumoxids die hervorragenden Eigenschaften erreicht werden. Unter Reinheit ist dabei zu verstehen, dass in dem Aluminiumoxid möglichst wenig Fremdstoffe als weitere Bestandteile sein sollen, die zu einer glasartigen Zwischen- oder Übergangsphase führen könnten. Nicht im Widerspruch zu dieser Forderung steht, dass dem Ausgangspulver, also dem Aluminiumoxid bestimmte Zusätze, wie beispielsweise Magnesiumoxid, als Kornwachstumshemmer zugesetzt werden können.

Zur Erprobung der neuen Hüftkopfkappenkonzeption wurden sowohl Tier- als auch Leichenversuche durchgeführt. Nach der Darstellung des Hüftgelenkes wurde parallel zur Femurmittelachse eine Bohrung durch den Pol des Hüftkopfes angebracht. Diese Bohrung diente zur Führung des Zentrierstiftes einer oszillierenden Hohlfräse, mit der das zylindrische Implantatlager vorbereitet wurde. Im Winkel von 90° zur Zylindermittelachse wurde dann ein Kugelsegment reseziert, wobei die Resektion durch einen rotierenden, in der Bohrung geführten Stirnfräser nach Art eines Zapfensenkers erfolgte. Die Oberkanten des entstandenen Knochenzylinders wurden mit einem ebenfalls mit einem Zentrierstift in der Bohrung geführten Instrument verrundet. Danach erfolgte das Aufsetzen der Kappe mit Presspassung. Der Leichenversuch zeigte, dass die Hüftkopfkappe für den Einsatz beim Humanpatienten grosse Vorteile aufweist, da nur eine minimale Resektion erforderlich war, eine gute Gefässversorgung gewährleistet werden konnte und eine feste Primärfixation ohne Knochenzement erreicht wurde. Nach der Reposition wurde die Funktion des Hüftgelenkes überprüft. Es traten weder Behinderungen noch Luxationen auf.

Der Tierversuch wurde mit Foxhounds durchgeführt, die 14 Tage nach der Operation ein zunehmend unauffälliger werdendes Gangbild aufwiesen. Sie erholten sich schnell und konnten 2 Monate später in die Zwingeranlage zurückgebracht werden, wo sie in der natürlichen Umgebung der Meute eine grosse Bewegungsfreudigkeit zeigten. Sie konnten sich nach der Operation voll in der Meute behaupten, was ebenso wie die Röntgenaufnahme die volle Funktionsfähigkeit und die problemlose Einheilung der eingesetzten Hüftkopfkappe aus hochreiner Aluminiumoxidkeramik bestätigte.

Die Erfindung wird nachstehend an Hand der Zeichnungen erläutert:

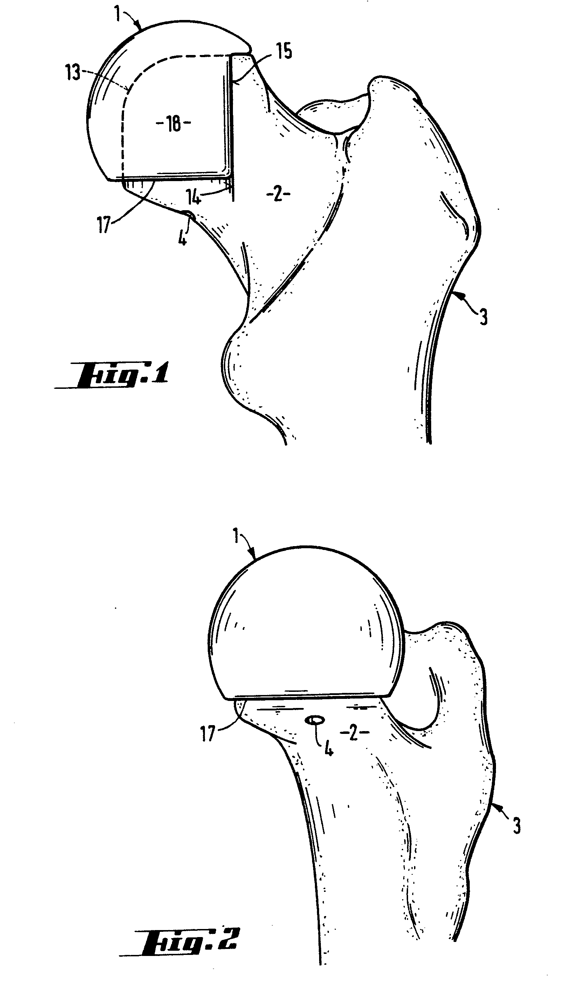

Fig. 1 zeigt ein proximales Femurende mit aufgesetzter Hüftkopfkappe,

Fig. 2 das gleiche proximale Femurende mit aufgesetzter Hüftkappe, wobei der Femur jedoch um ca. 45° gedreht wurde,

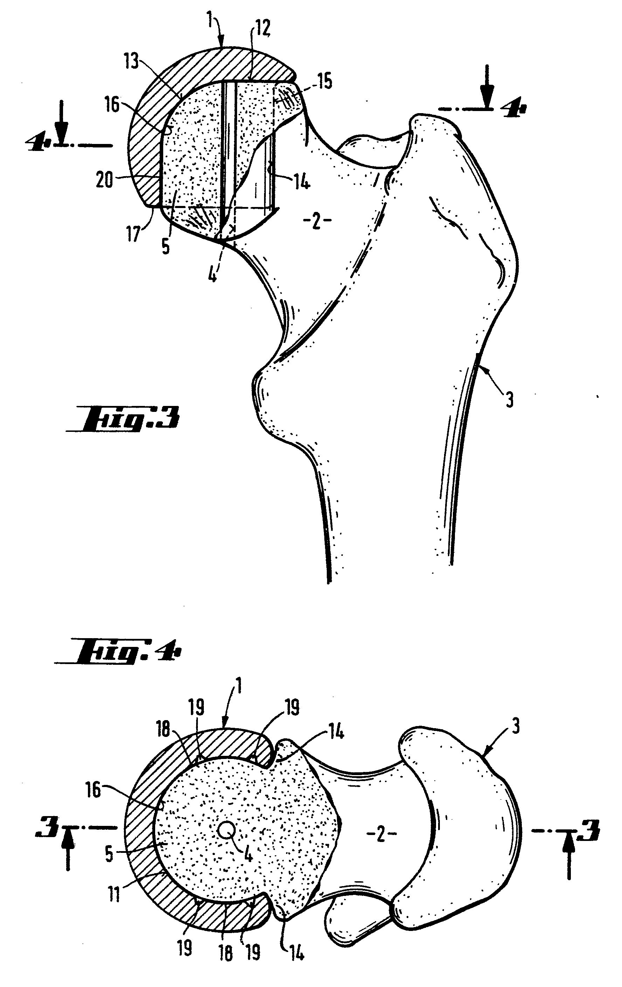

Fig. 3 einen Medialschnitt durch den resezierten Femurkopf, parallel zur Femurachse geschnitten, mit aufgesetzter Hüftkappe,

Fig. 4 den Schnitt durch den resezierten Femurkopf mit aufgesetzter Hüftkappe im 90°-Winkel zur Femurachse geschnitten,

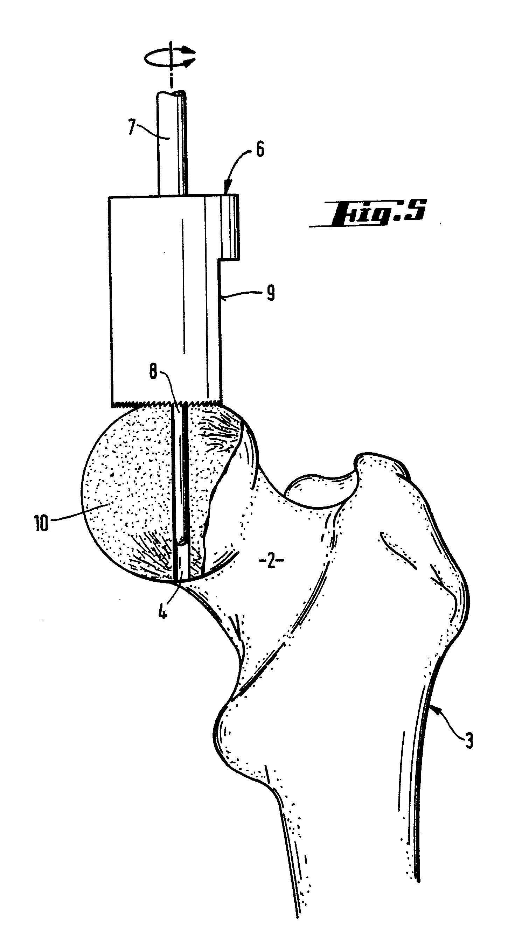

Fig. 5 ein Resektionsschema mit oszillierendem Hohlfräser,

Fig. 6 bis 9 zeigen Schnitte durch eine Hüftkopfkappe.

Durch einen nicht dargestellten Bohrer wird in den Hüftkopf 10 das Bohrloch 4 eingebracht, das zur Führung des Zentrierstiftes 8 des Hohlfräsers 6 dient. Der Hohlfräser 6 weist einen Ansatz 7 auf, an den eine nichtdargestellte oszillierende Säge angreift, die den Hohlfräser 6 entlang eines Kreisbogens 11 bewegt. Analog wird durch den Einsatz eines nichtdargestellten Stirnfräsers, der ebenfalls im Bohrloch 4 mittels eines Zentrierstiftes 8 geführt wird, die Kopffläche des Hüftkopfes 10 abgearbeitet.

Bei der Resektion des Hüftkopfes 10 mittels des Hohlfräsers 6 entstehen am Ende des Kreisbogens 11 Anschläge 14, durch die ein Verdrehen der Hüftkopfkappe 1 vermieden wird. Die torartige Öffnung 15 des kuppelförmigen Hohlraumes 16 in der Hüftkopfkappe 1 ist gegen den Schenkelhals 2 des Femurs 3 gerichtet, d.h. dass der resezierte Hüftkopfstumpf 5 sowohl im Bodenbereich 17 der Hüftkopfkappe 1 als auch im Bereich des Schenkelhalses 2 keine Gefässbeschädigungen aufweist. Um die Beschädigung der Gefässe am Schenkelhals 2 zu vermeiden, ist der Hohlfräser 6 mit einer Aussparung 9 versehen und wird nur durch eine geringfügig oszillierende Bewegung gedreht, um den Kreisbogen 11 zu bilden. Nach der Bildung der Rundung 13 kann die Hüftkopfkappe 1 aufgepresst werden, die dadurch ihren endgültigen Sitz erhält. Sie weist an ihren Stirnflächen 18 Nuten 19 auf, in die Knochenmaterial einwachsen kann. Die Stirnfläche 20 weist keine Nuten 19 auf, um die Festigkeit der Hüftkopfkappe 1 nicht zu reduzieren.

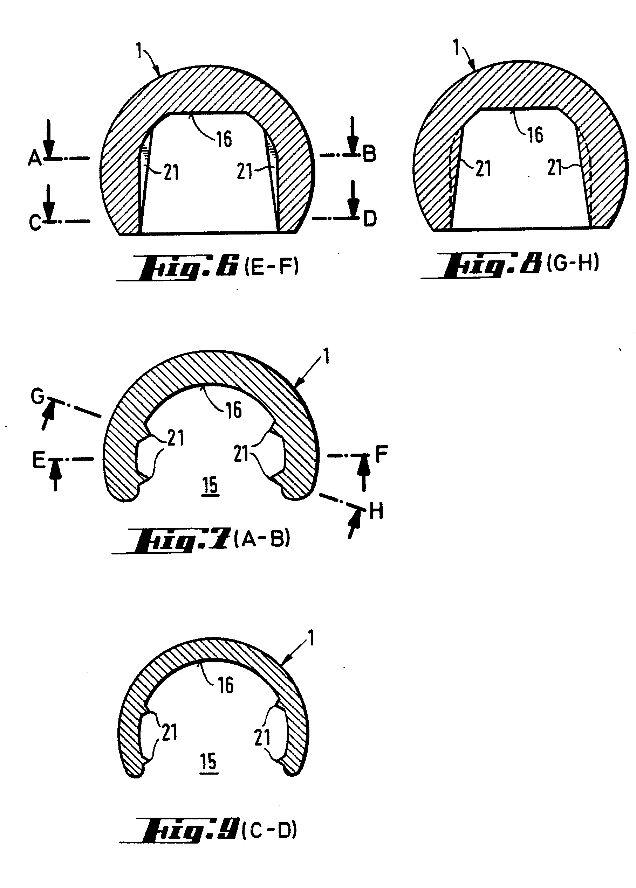

In den Fig. 6 bis 9 sind Schnitte durch eine Hüftkopfkappe 1 dargestellt, die konisch verlaufende Rippen 21 aufweist. Die konischen Rippen 21 besitzen dabei eine dreikantige Form. Sie können durch Pressen in den resezierten Hüftkopfstumpf 5 eingedrückt werden. Die Primärbefestigung wird durch diese Rippen erhöht, und die Hüftkopfkappe 1 damit noch rotationsstabiler verankert.

## Patentansprüche

1. Hüftkopfkappe als Endoprothese, die aussen im wesentlichen kuppelförmig ausgebildet ist und zur Aufnahme des bearbeiteten Femurkopfes (10) einen im wesentlichen rotationssymmetrischen Hohlraum (16) mit zum Schenkelhals (2) weisender Öffnung (15) enthält, dadurch gekennzeichnet, dass die Rotationsachse des Hohlraumes (16) der Hüftkopfkappe (1) im Einbauzustand etwa parallel zur Femurmittelachse verläuft und dass der Hohlraum (16) den resezierten Hüftkopfstumpf (5) um mehr als 180 Grad umgreift und eine U-förmig gestaltete zum Schenkelhals (2) weisende Öffnung (15) besitzt.

2. Hüftkopfkappe nach Anspruch 1, dadurch gekennzeichnet, dass der kuppelförmige Hohlraum (16) in der Hüftkopfkappe (1) Zylinderform hat.

3. Hüftkopfkappe nach Anspruch 1, dadurch gekennzeichnet, dass der kuppelförmige Hohlraum (16) in der Hüftkopfkappe (1) ein Kegelstumpf ist.

4. Hüftkopfkappe nach einem der Ansprüche 1 bis 3, dadurch gekennzeichnet, dass sie mittels Presssitz mit dem Hüftkopfstumpf (5) verbunden ist.

5. Hüftkopfkappe nach einem der Ansprüche 1 bis 4, dadurch gekennzeichnet, dass die Innenfläche der Hüftkopfkappe (1) mit Nuten (19) versehen ist.

6. Hüftkopfkappe nach einem der Ansprüche 1 bis 5, dadurch gekennzeichnet, dass die Innenfläche der Hüftkopfkappe (1) mit Rippen (21) versehen ist.

7. Hüftkopfkappe nach einem der Ansprüche 1 bis 6, dadurch gekennzeichnet, dass die Innenfläche der Hüftkopfkappe (1) eine rauhe Oberfläche besitzt.

8. Hüftkopfkappe nach einem der Ansprüche 1 bis 7, dadurch gekennzeichnet, dass die Innenfläche der Hüftkopfkappe (1) mit Poren versehen ist, deren Grösse mehr als $80\,\mu$ beträgt.

9. Hüftkopfkappe nach einem der Ansprüche 1 bis 8, dadurch gekennzeichnet, dass die Hüftkopfkappe (2) aus hochreiner Oxidkeramik besteht.

10. Hüftkopfkappe nach einem der Ansprüche 1 bis 9, dadurch gekennzeichnet, dass sie aus einem gesinterten Aluminiumoxid mit einer Dichte gleich oder grösser $3{,}92\ \text{g/cm}^3$, einer Porosität gleich oder kleiner 2%, einer Wasseraufnahme gleich oder kleiner 0,01%, einer Reinheit gleich oder grösser 99,7% $Al_2O_3$, einer Vickershärte (P=2M) gleich oder grösser 22 000 $N/mm^2$, einer mittleren Korngrösse gleich oder kleiner $10\,\mu$, einer mittleren Biegefestigkeit gleich oder grösser 300 $N/mm^2$, einer Druckfestigkeit gleich oder grösser 160 $N/mm^2$ besteht.

## Claims

1. Hip-joint cap as an endoprothesis the outside of which is substantially dome-shaped and which contains a substantially rotationally symmetrical cavity (16) for accommodating the prepared head (10) of the femur which cavity has an opening (15) facing the neck (2) of the femur, characterised in that, when the cap has been fitted, the axis of rotation of the cavity (16) of the hip-joint cap (1) runs approximately parallel to the central axis of the femur and that the cavity (16) encompasses the resected stump of the hip-joint (5) by more than 180° and has an opening (15) that is U-shaped and faces the neck (2) of the femur.

2. Hip-joint cap according to claim 1, characterised in that the dome-shaped cavity (16) in the hip-joint cap (1) is cylindrical.

3. Hip-joint cap according to claim 1, characterised sed in that the dome-shaped cavity (16) in the hip-joint cap (1) is a truncated cone.

4. Hip-joint cap according to one of claims 1 to 3, characterised in that it is force-fitted onto the hip-joint stump (5).

5. Hip-joint cap according to one of claims 1 to 4, characterised in that the inner face of the hip-joint cap (1) is provided with grooves (19).

6. Hip-joint cap according to one of claims 1 to 5, characterised in that the inner face of the hip-joint cap (1) is provided with ribs (21).

7. Hip-joint cap according to one of claims 1 to 6, characterised in that the inner face of the hip-joint cap (1) has a rough surface.

8. Hip-joint cap according to one of claims 1 to 7, characterised in that the inner face of the hip-joint cap (1) is provided with pores that are more than $80\,\mu$ in diameter.

9. Hip-joint cap according to one of claims 1 to 8, characterised in that the hip-joint cap (1) consists of high-purity oxide ceramics.

10. Hip-joint cap according to one of claims 1 to 9, characterised in that it consists of a sintered aluminium oxide having a density equal to or greater than $3.92\ \text{g/cm}^3$, a porosity equal to or less than 2%, a water absorption equal to or less than 0.01%, a purity equal to or greater than 99.7% $Al_2O_3$, a Vickers diamond pyramid hardness (P=2M) equal to or greater than 22 000 $N/mm^2$, a mean grain size equal to or less than $10\,\mu$, a mean bending strength equal to or greater than 300 $N/mm^2$, a compression strength equal to or greater than 4000 $N/mm^2$ and a tensile strength equal to or greater than 160 $N/mm^2$.

## Revendications

1. Bonnet pour tête de fémur comme endoprothèse, qui extérieurement est conçu particulier en forme de cupule et contient une cavité (16), pour l'essentiel symétrique de révolution, destinée à recevoir la tête (10) du fémur façonnée, et comportant une ouverture (15) pointant vers le col (2) du fémur, caractérisé par le fait que l'axe de rotation de la cavité (16) du bonnet (1) pour tête de fémur est, une fois mis en place, sensiblement parallèle à l'axe médian du fémur et que la cavité (16) entoure la partie restante (5) de la tête du fémur réséquée sur plus de 180°, et possède une ouverture (15) façonnée en forme de U, pointant vers le col (2) du fémur.

2. Bonnet pour tête de fémur selon la revendication 1, caractérisé par le fait que la cavité (16) en forme de cupule dans le bonnet (1) pour tête de fémur a une forme cylindrique.

3. Bonnet pour tête de fémur selon la revendication 1, caractérisé par le fait que la cavité (16) en forme de cupule dans le bonnet (1) pour tête de fémur est un tronc de cône.

4. Bonnet pour tête de fémur selon l'une quelconque des revendications 1 à 3, caractérisé par le fait qu'il est assemblé avec la partie restante (5) de la tête du fémur à l'aide d'un ajustement pressé.

5. Bonnet pour tête de fémur selon l'une quelconque des revendications 1 à 4, caractérisé par le fait que la surface intérieure du bonnet (1) pour tête de fémur est munie de gorges (19).

6. Bonnet pour tête de fémur selon l'une quelconque des revendications 1 à 5, caractérisé par le fait que la surface intérieure du bonnet (1) pour tête de fémur est munie de nervures (21).

7. Bonnet pour tête de fémur selon l'une quelconque des revendications 1 à 6, caractérisé par le fait que la surface intérieure du bonnet (1) pour tête de fémur possède une surface rugueuse.

8. Bonnet pour tête de fémur selon l'une quelconque des revendications 1 à 7, caractérisé par le fait que la surface intérieure du bonnet (1) pour tête de fémur est munie de pores dont la grosseur est supérieure à 80 $\mu$.

9. Bonnet pour tête de fémur selon l'une quelconque des revendications 1 à 8, caractérisé par le fait que le bonnet (1) pour tête de fémur est constitué par une céramique d'oxydes très pure.

10. Bonnet pour tête de fémur selon l'une quelconque des revendications 1 à 9, caractérisé par le fait qu'il est constitué par un oxyde d'aluminium fritté ayant un poids spécifique égal ou supérieur à 3,92 g/cm$^3$, une porosité égale ou inférieure à 2%, une absorption d'eau égale ou inférieure à 0,01%, une pureté égale ou supérieure à 99,7% d'Al$_2$O$_3$, une dureté Vicker (P=2M) égale ou supérieure à 22 000 N/mm$^2$, une grosseur moyenne des grains égale ou inférieure à 10 $\mu$, une résistance moyenne à la flexion égale ou supérieure à 300 N/mm$^2$, une résistance à la compression égale ou supérieure à 4000 N/mm$^2$ et une résistance à la traction égale ou supérieure à 160 N/mm$^2$.

**Fig. 1**

**Fig. 2**

*Fig. 3*

*Fig. 4*

Fig. 5

*Fig. 6* (E-F)

*Fig. 8* (G-H)

*Fig. 7* (A-B)

*Fig. 9* (C-D)